# EUROPEAN PATENT SPECIFICATION

(11) **EP 0 748 225 B1**
(45) Date of publication and mention of the grant of the patent: **09.06.2004**
(21) Application number: 95911035.4
(22) Date of filing: 28.02.1995
(51) Int. Cl.: A61K 38/46, C12N 9/96, A61K 9/12

(54) **PHARMACEUTICALLY ACCEPTABLE DNase FORMULATION**
PHARMAZEUTISCHE AKZEPTABEL DNASE ZUSAMMENSETZUNG
FORMULATION DE DNase PHARMACEUTIQUEMENT ACCEPTABLE

(30) Priority: 04.03.1994 US 206020; 27.12.1994 US 364074
(43) Date of publication of application: 18.12.1996
(73) Proprietor: GENENTECH, INC., South San Francisco California 94080 (US)
(72) Inventor: CHAN, Hak-Kim, San Mateo, CA 94402 (US); CLARK, Andrew, R., Half Moon Bay, CA 94019 (US); GONDA, Igor, San Francisco, CA 94109 (US); HSU, Chung, C., Los Altos Hills, CA 94022 (US); MUMENTHALER, Marco, CH-4132 Muttenz (CH); SHIRE, Steven, J., Belmont, CA 94002 (US)
(74) Representative: Plougmann & Vingtoft A/S
(86) International application number: PCT/US1995/002041
(87) International publication number: WO 1995/023613

(56) References cited:
- EP-A- 0 501 375
- WO-A-90/07572
- WO-A-91/18091
- WO-A-93/25670
- US-A- 4 233 405
- NETHERLANDS MILK AND DAIRY JOURNAL, vol. 23, 1969 DUIVENDAAL, NL, pages 46-54, M.J. VAN DE BEEK ET AL 'Preservation of the enzymatic activity of renin during spray drying and during storage, and the effect of sugars and certain other additives'
- S.T.P. PHARMACEUTICAL SCIENCES, vol. 4,no. 1, February 1994 PARIS, pages 50-62, CIPOLLA, D.C. ET AL 'Assessment of aerosol delivery systems for recombinant human deoxyribonuclease'

## Description

### Field of the Invention

The present invention is related to results obtained from research on the formulation of deoxyribonuclease, otherwise referred to as DNase, a phosphodiesterase that is capable of hydrolyzing polydeoxyribonucleic acid (DNA).

The present invention relates generally to the preparation of pharmaceutically acceptable formulations comprising spray-dried DNase in therapeutically effective form for administration into the lung of an individual. It relates to these formulations per se and to their use clinically and to methods of preparing and using such spray-dried DNase formulations.

### Background of the Invention

DNase is a phosphodiesterase capable of hydrolyzing polydeoxyribonucleic acid. DNase has been purified from various species to various degrees. The complete amino acid sequence for a mammalian DNase was first made available in 1973. See, e.g., Liao, et al., J. Bio. Chem, 248, 1489 (1973).

DNase has a number of known utilities and has been used for therapeutic purposes. Its principal therapeutic use has been to reduce the viscoelasticity of pulmonary secretions in such diseases as pneumonia and cystic fibrosis, thereby aiding in the clearing of respiratory airways. See, e.g., Lourenco, et al., Arch. Intern. Med. 142, 2299 (1982); Shak, et al., Proc. Nat. Acad. Sci. 87, 9188 (1990); and Hubbard, et al., New England Journal of Medicine 326, 812 (1992).

DNA encoding human DNase has been isolated and sequenced and that DNA has been expressed in recombinant mammalian host cells, thereby enabling the production of human DNase in commercially useful quantities. See, e.g., WO 90/07572 or Shak, et al., Proc. Nat. Acad. Sci. 87, 9188 (1990). Recombinant human DNase (rhDNase) has been found to be useful clinically, especially in purified form such that the DNase is free from proteases and other proteins with which it is ordinarily associated in nature.

The means and methods by which human DNase can be obtained in pharmaceutically effective form is described in the patent applications cited above. Various specific methods for the purification of DNase are known in the art. See, e.g., Khouw, et al., U.S. Patent No. 4,065,355, issued 27 December 1977; Markey, FEBS Letters 167, 155 (1984); and Nefsky, et al., Euro. Journ. Biochem. 179, 215 (1989).

The present application is predicated on the use of spray-dried DNase for formulation. DNase can be employed as such, as a mixture of deamidated and non-deamidated forms, or in isolated deamidated and non-deamidated forms, non-deamidated human DNase being regarded as the more active species. The preparation and separation of such forms are the subject matter of the patent application cited above.

The present invention is directed to the formulation of spray-dried DNase (including all of its biologically active forms as previously noted) such that the biologically active species can be administered directly into the lung for therapeutic effect.

Because DNase exhibits its therapeutic effect in the lungs of an individual, it is essential that the DNase be deposited in the respiratory tract in a therapeutically active form. Thus, it is important that formulations of DNase contain such DNase in substantially biologically active form, and in order to assure administration effectively, it is important that the DNase be formulated such that, when inhaled, it will be delivered in biologically active form preferably into the lung of the individual being treated.

Initially, the DNase was subjected to more or less standard lyophilization techniques in order to produce a dry powdered form that could be inhaled. The process of lyophilization is difficult to manage in order to consistently produce powders that exhibit properties satisfying the requirements of proper administration for therapeutic effect: dispersibility, uniform particle size, non-aggregated forms, biologically active drug principle.

Generally, spray-drying techniques have found a relatively wide range of applications within the chemical industry, the food industry and the biochemical and pharmaceutical industries. See, e.g., Drug Development and Industrial Pharmacy 18 (11 and 12), 1169-1206 (1992), entitled "The Spray Drying of Pharmaceuticals" and, generally, U.S. Patent No. 4,233,405.

Spray drying is becoming recognized as a method for the processing of materials; it has been used for pharmaceuticals such as antibiotics, and especially food products. The spray drying of pharmaceutical proteins is a new area of interest, and accordingly, has not been exploited commercially.

A number of problems attend the process of spray drying which could result in a product that does not have the required therapeutic characteristics as outlined above.

For example, Mumenthaler, et al. presented a feasibility study at the Sixth Annual AAPS Meeting in Washington, D. C. in November of 1991; that study was subsequently published in Pharmaceutical Research 11, 12 (1994). This feasibility study reported on endeavors to spray dry two protein pharmaceuticals: recombinant human growth hormone and recombinant tissue-type plasminogen activator. That study emphasized that the application of spray drying to therapeutic proteins is rather unexplored undoubtedly because of the concern that proteins may be thermally degraded during the operation, and hence, not suitably biologically active for therapeutic use. In this report, a number of publications are cited that would serve to confirm that prejudice in the art.

In the study, it was reported that although human growth hormone could be dried to a residual moisture content of about or less than 4%, approximately upwards of 25% of the protein was degraded during the processing resulting in a product that could not be exploited commercially for requisite therapeutic effect.

Contrary to what was found for human growth hormone, spray drying proved feasible with tissue plasminogen activator, emphasizing that unappreciated differences in molecular structure, and other factors influence the sensitivity of proteins to the phenomena essential for producing a therapeutically useful formulation by this means.

Thus, for inhalation or administration by inhalation into the lungs, these mixed results emphasize that it is not predictable that one could achieve success in producing a therapeutically acceptable formulation of a given biologically active protein in this manner. Further, the additional requirements needed for administration of proteins by therapeutic inhalation introduces further uncertainty.

The effect of excipients and of thermal stability are matters that complicate prediction as to therapeutic success. Indeed, commercial enterprises have been established to conduct further research on this means of producing therapeutic formulations.

### Summary of the Invention

The present invention is predicated on the finding that DNase together with a number of compatible excipients can be formulated via spray drying to produce a dispersion of dry powder containing the DNase as active drug principle in biologically active form, having the physical characteristics such that the formulation is suitable for administration into the lung of an individual for consequential therapeutic effect.

The present invention is thus directed to a method for the preparation of a pharmaceutically acceptable formulation comprising DNase which comprises the steps of spray drying a liquid composition-comprising compatible excipients and DNase as active drug principle and collecting the spray-dried product of such a step as a dispersible powder containing DNase in biologically active form.

The present invention is directed as well to the therapeutically effective DNase formulation resulting as a product of the process for its preparation herein.

The present invention is directed to such formulations, and their preparation, as well as all associated embodiments thereof relating to the preparation of formulations and their use therapeutically.

The present invention is more particularly directed to the preparation of pharmaceutically acceptable formulations comprising DNase as a powder to be used in an aerosol form, that is, a suspension of finely divided solid particles or solid particles in liquid droplets, suspended in a gas, that have proper dispersibility and particle size properties, preferably in substantially non-aggregated form, such that when administered into the lung of an individual they will be in a form likely to exhibit a therapeutic effect therein by the biologically active DNase drug principle.

The present invention is directed to the preparation of such formulations via a spray-drying technique having general parameters that when combined are useful for preparing the therapeutically active formulation. In particular, DNase powders can be produced in formulations in which less than about 4% of the active DNase principle is in aggregated form. The denaturation temperature of DNase is about 50 - 75°C depending on the medium. Thus, it is preferable to use inlet temperatures for the spray-drying process that do not exceed that temperature range by a great deal.

Similarly, the outlet temperature is an important consideration in respect of the denaturation temperature of DNase. It was determined that an outlet temperature in the spray-drying process as low as possible consistent with drying the product is preferable, if not optimal. Generally, an outlet temperature of from about ambient to 75°C is employed, and more preferably, from about ambient to 70°C.

In respect of pH of the solution that is subjected to spray drying, a range of from about 5 to 8 is probably optimal to avoid excessive aggregation and deamidation. It was found that ranges of pH of about 6 to 7 are preferred.

The spray-drying process employs initially a solution containing DNase. Such solutions preferably are aqueous and will contain one or a number of pharmaceutically compatible excipients. Concentrations of DNase are from about 1 to about 80 or more milligrams per milliliter, more preferably 5 to 30 milligrams per milliliter.

While a wide range of pharmaceutically acceptable excipients are available and could be used in producing solutions that are preferable for the spray-drying process herein, it was found that the characteristics of the excipient are important but not (necessarily) essential for producing a proper dispersible end product. In general, it has been found that suitable excipients are selected from the group consisting of sodium chloride, sugars (sucrose, lactose, trehalose and mannitol, for example), derived calcium or other divalent cations (such as can be obtained from calcium chloride, zinc chloride, manganese chloride and magnesium chloride to name a number of examples). The mass concentration in conjunction with droplet size controls the particle size. It has been found, for example, that increasing the amount of salt increases the dispersibility qualities of the spray-dried product. In the case of sugars, increasing their proportion over about 30% lowers dispersibility qualities.

The spray-drying process hereof can be used suitably with any of a number of devices that are available in the art and commercially.

The product powders hereof can be blended with additional known, dry excipients via usual procedures to produce blends thus tailored for various applications.

### Brief Description of the Drawings

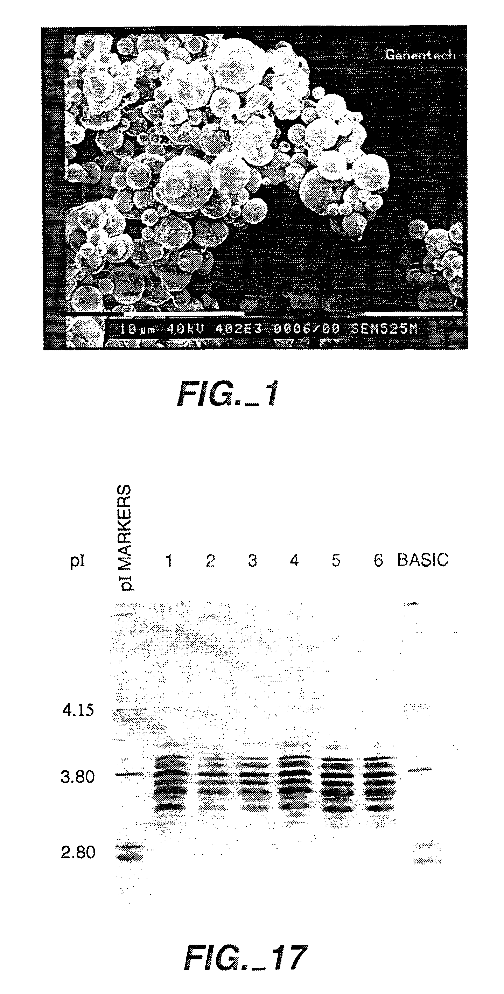

Figure 1 shows a scanning electron micrograph of rhDNase particles prepared as described in Example 1 hereof.

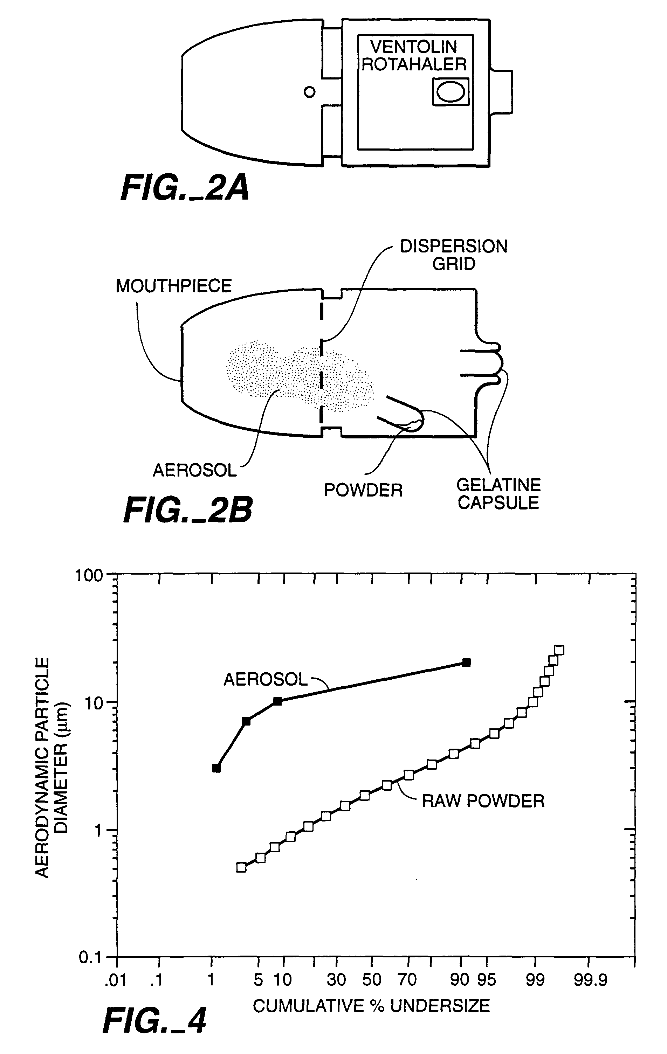

Figures 2A and 2B show two views of the Rotahaler® given as an example of a device used to redisperse the rhDNase powders.
Figure 3 is the multistage liquid impinger with the Rotahaler connected to the throat of the impinger for aerosol sizing of the rhDNase powders.
Figures 4 to 7 are comparisons of the particle size distribution of the raw powders to that in the aerosol cloud generated by the Rotahaler for four formulations containing different NaCl contents.
Figure 8 is a summary of the redispersibility versus particle size for rhDNase powders containing NaCl.
Figures 9A and 9B show two representations of the relationship between the primary particle size and the powder's redispersibility into respirable aerosol particles for a type of polydisperse powder (e.g., geometric standard deviation - 2.5).
Figure 10 shows the effect of NaCl content on the redispersion of the rhDNase powders.
Figure 11 shows a linear relationship between the NaCl content in the formulations and their crystallinity.
Figures 12A-12F show scanning electron micrographs of rhDNase particles containing NaCl.
Figure 13 is a comparison of the particle size distribution of the raw powder and the aerosol clouds generated by the Rotahaler for the rhDNase powder formulated with 40% lactose.
Figure 14 shows the redispersibility of rhDNase powders containing different sugar versus their sugar content.
Figure 15 shows the effect of particle size on the redispersibility of rhDNase powders formulated with sugar.
Figures 16A-16F show scanning electron micrographs of rhDNase particles containing sugars (16A,B,C: 40% sugar, 16D,E,F: 84% sugar; 16A,D: sucrose, 16B,E: lactose, and 16C,F: trehalose).
Figure 17 shows an isoelectric focusing gel for DNase-containing formulations. Lanes 1 and 4 are the DNase-salt formulation, lanes 2 and 5 are the DNase-lactose formulation and lanes 3 and 6 are the pure DNase formulation.

### Detailed Description of the Invention

### A. Definitions

By the term "DNase" or "human DNase" or "recombinant human DNase" herein is meant a polypeptide having the amino acid sequence of human mature DNase as well as amino acid sequence variants thereof (including allelic variants) that are enzymatically active in hydrolyzing DNA. Thus, the terms herein denote a broad definition of those materials disclosed and prepared in the various patent applications cited above and incorporated herein by reference. It will be understood that the terms include both purified mixtures of deamidated and non-deamidated human DNase as well as purified forms of each.

By the term "excipient" herein is meant a pharmaceutically acceptable material that is employed together with DNase for the proper and successful preparation of a spray-dried formulation that results in therapeutic effect when administered into the lung of an individual patient. Suitable excipients are well-known in the art, and are generally described above and, for example, in the Physician's Desk Reference, the Merck Index and Remington's Pharmaceutical Sciences.

By the term "therapeutically effective" and grammatical equivalents thereof herein is meant dosages of from about 1 microgram to about 1 milligram of human DNase per kilogram of body weight of the individual being treated, administered within the spray-dried pharmaceutical formulations hereof. The current daily therapeutic dose of DNase is about 2.5 mg. The therapeutically effective amount of human DNase will depend, for example, upon the therapeutic objectives and the condition of the individual being treated. In all of that, the present invention provides as an essential component, spray-dried formulations containing therapeutically effective amounts, the formulations being prepared such that they suitably provide such therapeutic effect when administered into the lung of the individual.

The term "aerosol" herein refers to the use of the term in a pharmaceutical sense, and covers formulations that are comprised of a suspension of fine solid particles in air or solid particles in liquid droplets or droplets of solution in air. The term includes substances dispensed from a container as an aerosol, particularly for administration of the contents into the lung of an individual by inhalation and covers the container for this act as well. The aerosols hereof are comprised of dispersed solid particles with a particle size suitable for introduction into the lung. The particles should be approximately 1 to 6 microns in size of substantially non-aggregated biologically active DNase molecules for achieving therapeutic effect into the lung of the individual being treated.

The DNase formulations hereof are employed for enzymatic alteration of the viscoelasticity of mucus within the lung. Such formulations are particularly useful for the treatment of patients with pulmonary disease who have abnormal viscous, purulent secretions and conditions such as acute or chronic bronchial pulmonary disease, including infectious pneumonia, bronchitis or tracheobronchitis, bronchiectasis, cystic fibrosis, asthma, tuberculosis and fungal infections and the like. For such therapies, the novel formulations hereof are instilled in otherwise conventional fashion into the bronchi of the individual being treated. The formulations hereof are particularly suited for the assured introduction into the lung of DNase such that a therapeutically effective amount of DNase is delivered to the individual by direct action in the lung.

### B. Preferred Embodiments

### Introduction

Dry powders of DNase were prepared for aerosol delivery. The powders were obtained by the method of spray drying; up to 4% of molecular aggregates were found in the powders. The presence of aggregates, in view of their potential for unwanted effects such as toxicity and immunogenicity, is preferably minimized. It is unlikely that the aggregation was due to the mechanical shearing stress during the process of spraying since DNase was not denatured during jet nebulization. It may be therefore related to the drying process during which the spray droplets were exposed to air at elevated temperature. In fact, previous experiments show that during ultrasonic nebulization, formation of DNase aggregates occurs due to thermal denaturation.

### C. Examples

### Example 1

An initial feasibility study designed to determine the particle size distribution and quality of the rhDNase after spray drying was undertaken using rhDNase at 4 mg/mL formulated in 150 mM NaCl and 1 mM CaCl₂ at pH6.6. The spray drying was performed using a laboratory scale spray-dryer (Büchi,Model 190). During operation, protein solution was fed at a constant rate of 5 mL/min with a peristaltic pump to a nozzle (0.5-mm I.D.), where atomization occurred by means of a pressurized air stream. Drying air entered the drying chamber at 90 °C and 36000L/hr (STP condition) in the same direction as the descending spray droplets. In order to avoid the aspiration of dust particles from the laboratory atmosphere, drying air and atomization air were prefiltered with 70- and 0.22-µm filters, respectively. The product powders were clarified from the drying-air stream by means of a cyclone and collected in a receiving vessel.
The resulting fine white spray dried powder was assayed for moisture content by thermal gravimetric analysis and particle size and morphology were determined by electron microscopy. The spray dried rhDNase powder (0.13 g) was also dissolved into 10 mL of milli-Q water, and the following assays were used to assess the quality of the redissolved rhDNase.
- color and clarity; to determine visual appearance of the solution.
- pH of reconstituted solution.
- UV spectrophotometric scan: to determine rhDNase concentration using an absorptivity of 1.6 (mg/mL)⁻¹ cm⁻¹.
- Gel sizing chromatography: to determine the presence of molecular aggregates and fragments of rhDNase.
- Atomic absorption flame photometry: to determine Na⁺ content.
- Isoelectric focusing (IEF): to determine charge distribution of rhDNase.
- SDS polyacrylamide gel electrophoresis (SDS PAGE): to determine presence of covalent aggregates and fragments of rhDNase.
- Methyl green DNA activity assay: to determine activity of rhDNase.

Results: The spray dried powders were found to contain approximately 30% rhDNase, 60% NaCl and 5% water. The molar ratio of sodium to protein in solution was maintained in the spray dried powder (Table A). The charge distribution of rhDNase was unaltered after spray drying as determined by IEF. The pH and color and appearance of the redissolved spray dried powder was essentially unchanged (there were a few particles observed in the clear reconstituted solutions). Gel sizing chromatography did show a slight decrease in the percent of monomer which was attributed to formation of aggregates. Some of the aggregates that are generated are due to intramolecular disulfide cross-linking shown by the presence of reducible high molecular weight bands detected by SDS PAGE. Most importantly the specific activity of the rhDNase after spray drying was unaltered (Table A). Electron microscopy of the spray dried powder showed spherical particles with a size range that suggests if dispersed properly and inhaled, these particles would likely be deposited in the broncho-tracheal region of the lungs (Figure A).

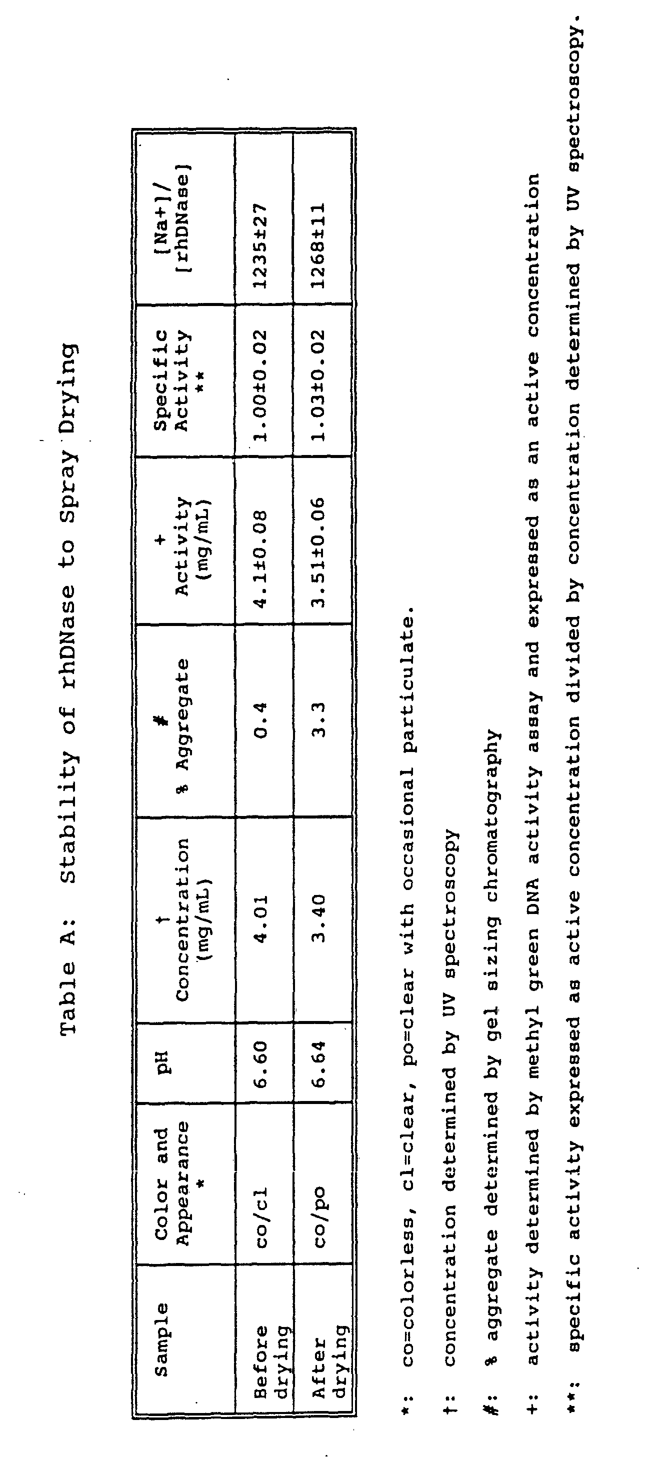

### Example 2

### Materials and Methods

rhDNase powders were prepared from a stock solution containing 4.7 mg/ml rhDNase, 1mM CaCl₂ and 150 mM NaCl. The stock solution was diafiltered to between 90 and 100 mg/ml rhDNase without water replenishment. Predetermined amounts of water and NaCl were added to the concentrated stock solution to prepare rhDNase solutions with a range of NaCl concentrations. For the sugar formulations, the solution was first dialyzed in highly purified water to remove excess NaCl and CaCl₂, then concentrated by diafiltration, followed by adjustment to the desired concentration of rhDNase and sugars. Details of the rhDNase and excipient contents in the powders are presented in Tables 1 and 2.

**Table 1.**

| Content of Spray Dried Powders of rhDNase with NaCl | | | | | |
|---|---|---|---|---|---|
| DNase%¹ | Water%² | NaCl%³ | Total% | Monomer%⁴ | Activity%⁵ |
| 10.46 | 1.3 | 87.6 | 99.4 | 96.5 | ND |
| 35.3 | 4.5 | 59.9 | 99.7 | 98.2 | 114^{a} |
| 47.6 | 5.5 | 45.2 | 98.3 | 99.5 | ND |
| 68.1 | 4.6 | 27.2 | 99.9 | 99.7 | ND |
| 82.2 | 10.1 | 8.00 | 100.1 | 99.5 | 105^{b} |

| | | | | | |
|---|---|---|---|---|---|
| Notes: 1. rhDNase content determined by UV absorption at 280 nm using an absorptivity of 1.6 (mg/ml)⁻¹cm⁻¹. | | | | | |
| 2. Water content determined by thermogravimetry. | | | | | |
| 3. NaCl content determined by flame photometry. | | | | | |
| 4. Monomer % was determined by size exclusion chromatography. | | | | | |
| 5. Activity was determined by the methyl green assay normalized to the protein concentration measured by UV absorption. ND: not determined. | | | | | |
| a: 114 ± 2%, number of determination = 8. | | | | | |
| b: The mean of two determined values of 109.8 and 100.7%. | | | | | |

**Table 2.**

| Actual Content of Spray Dried Powders of rhDNase with Sugars | | | |
|---|---|---|---|
| Excipient | DNase% | Water%¹ | Sugar %² |
| Sucrose | 11.2 | 6.62 | 82.2 |
| | 36.9 | 5.97 | 57.1 |
| | 70.5 | 6.08 | 23.4 |
| Lactose | 10.9 | 6.93 | 82.2 |
| | 35.4 | 6.24 | 58.4 |
| | 64.1 | 8.16 | 27.7 |
| | 85.8 | 5.98 | 8.2 |
| Trehalose | 11.2 | 7.48 | 81.3 |
| | 35.4 | 7.64 | 57.0 |
| | 68.1 | 8.10 | 23.8 |

| | | | |
|---|---|---|---|
| Notes: 1. At similar excipient weight proportion of approximately 30%, the sugar powders gave 1 to 3% higher moisture content than the NaCl powders. | | | |
| 2. Sugar content determined by weight difference. | | | |

Moisture content was determined thermogravimetrically (TGA 7, Perkin Elmer). Crystallinity of the powders was measured by X-ray powder diffraction. Particle morphology was observed under a scanning electron microscope (SEM) (525M, Philips).

### Spray Drying of rhDNase

Spray drying was carried out on a Büchi 190 Mini Spray Dryer using filtered and/or dehumidified air. In order to minimize possible thermal degradation of the products, the cyclone and collection vessel were cooled. The rhDNase solutions were delivered at a rate of 5 ml/min. The inlet and outlet air temperatures were 90 and 55°C, respectively.

### Results and Discussion

### Spray Dried Powders of rhDNase With Sodium Chloride

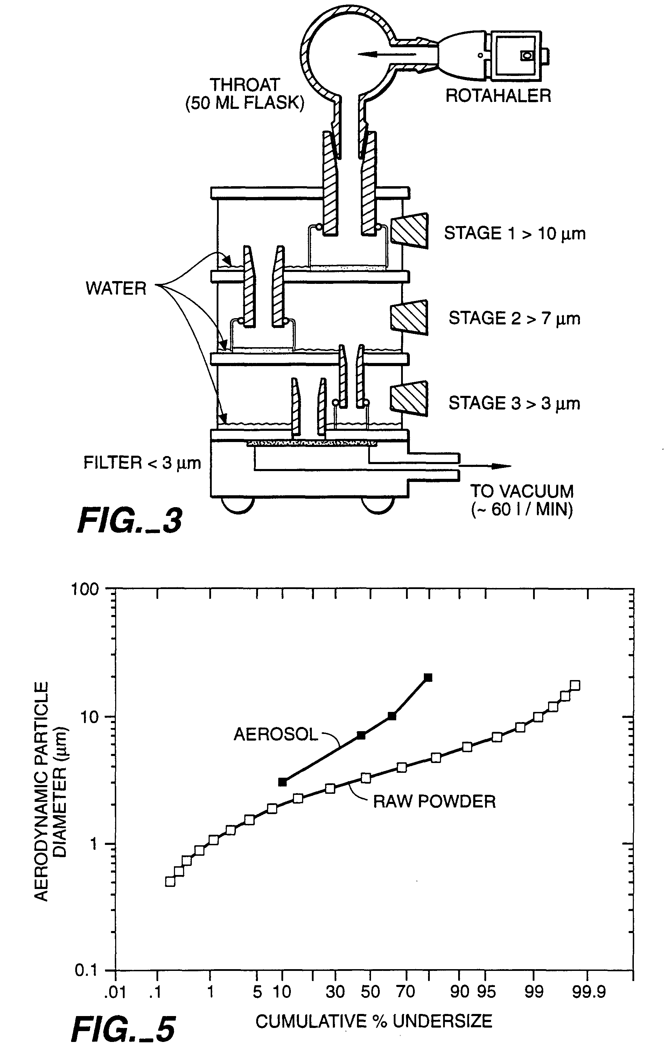

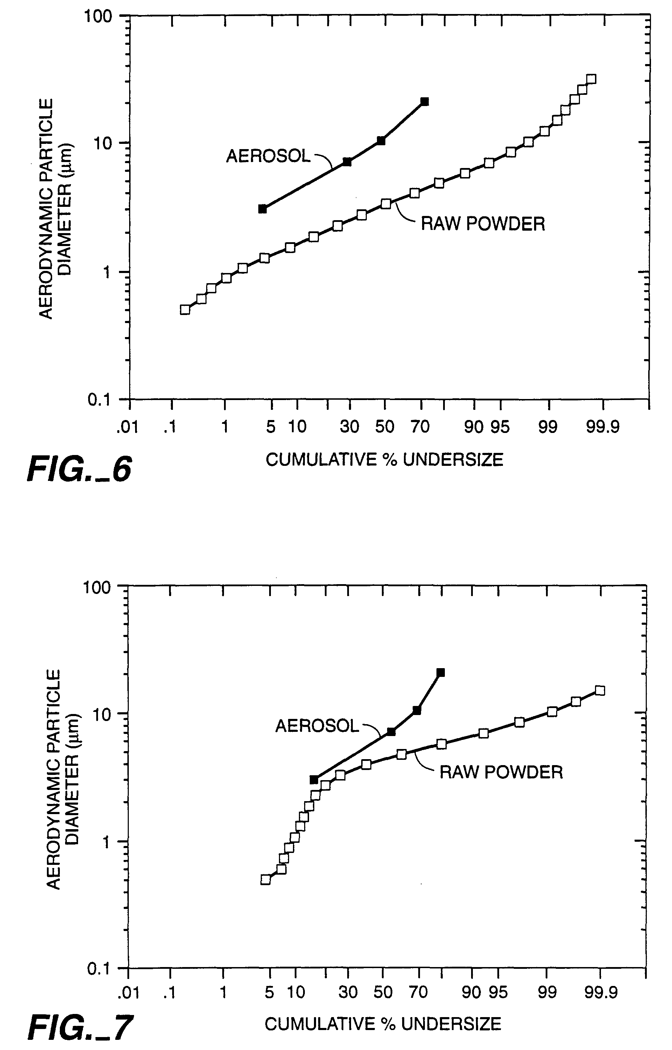

Figures 4-7 compare the particle size distribution of the original powder (as determined by laser diffraction) to that in the aerosol clouds generated by the Rotahaler (as determined by the multiple stage liquid impinger) for four formulations containing different salt contents. For the raw powders, the diameters were obtained assuming unit density. Correction using the measured density of 1.3 would slightly shift the curve upward but the effect is minimal since the aerodynamic diameter of a particle is proportional to the square root of its density. The data indicate that the aerosols were not sufficiently redispersed to recover the size distribution of the original particles. This is *common* for most powder inhalers and is a result of the powder cohesiveness and the dispersing efficiency of the device (Rotahaler).

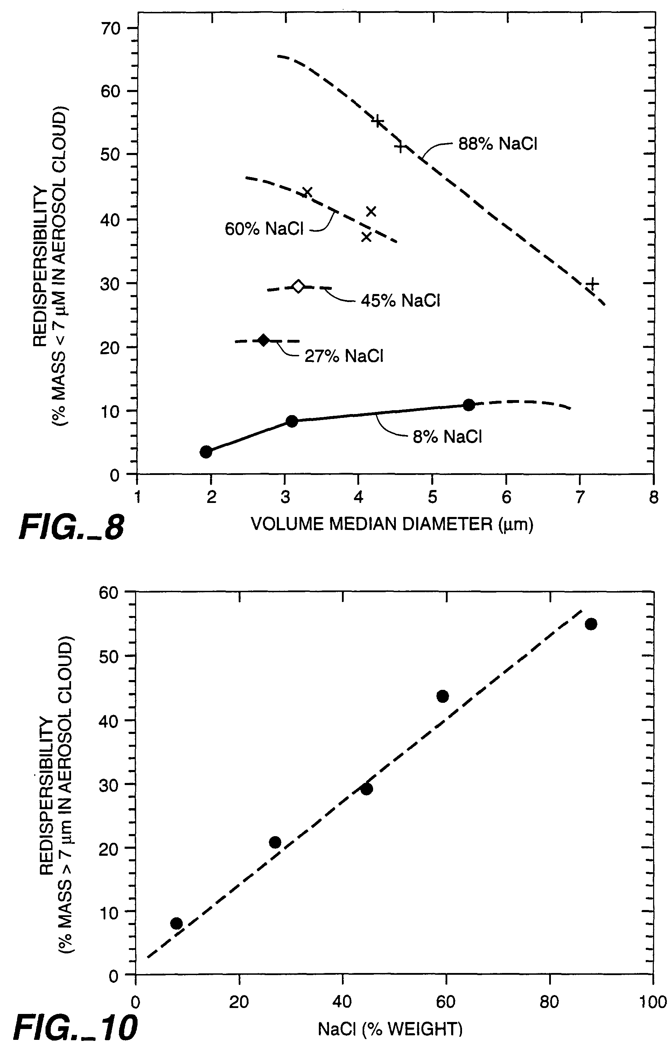

A summary of the redispersion as a function of the particle size and the NaCl (or rhDNase) content is presented in Figure 8. The extrapolated dash lines indicate the expected trend for the behavior of the powders.

### Effect of Particle Size

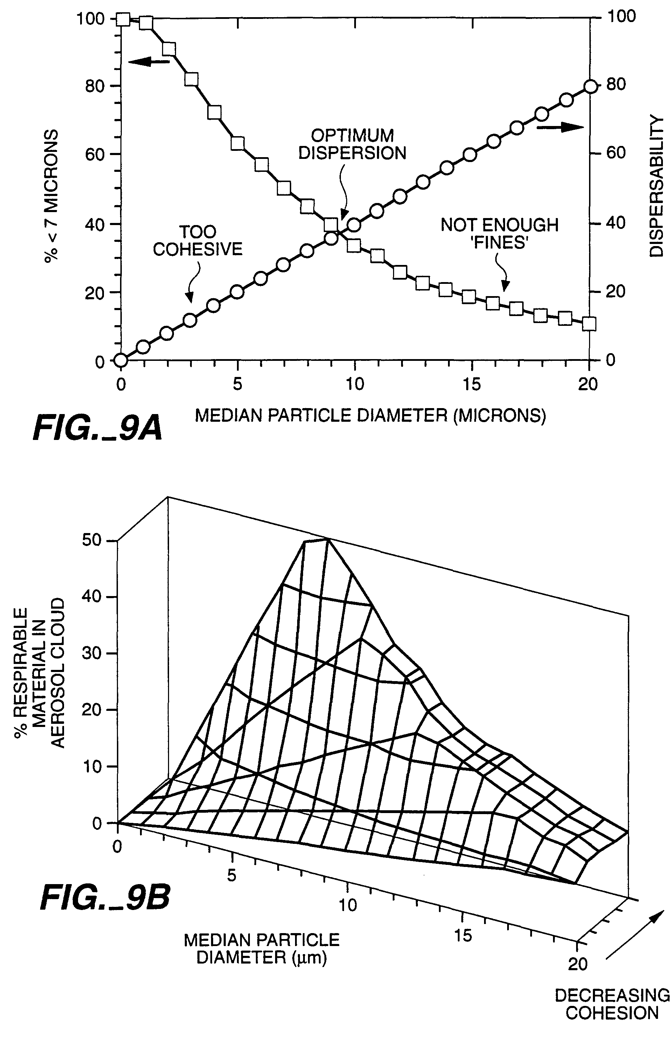

The expected relationship between the primary particle size and the redispersibility into respirable aerosol particles is illustrated in Figures 9A and 9B. Generally, the finer the particles the more cohesive they are, which would lead to poor redispersibility. As the particles become bigger, they are better dispersed due to a decrease in cohesiveness. On further increase in particle size, the redispersibility would reach a maximum and decrease again due to a decrease in the number of fine particles. The 8% NaCl formulation follows this behavior (Figure 8). It shows that the redispersibility improves from 4 to 10% as the mean particle size increases from 2 to 5.8 µm.

### Effect of the Excipient Content

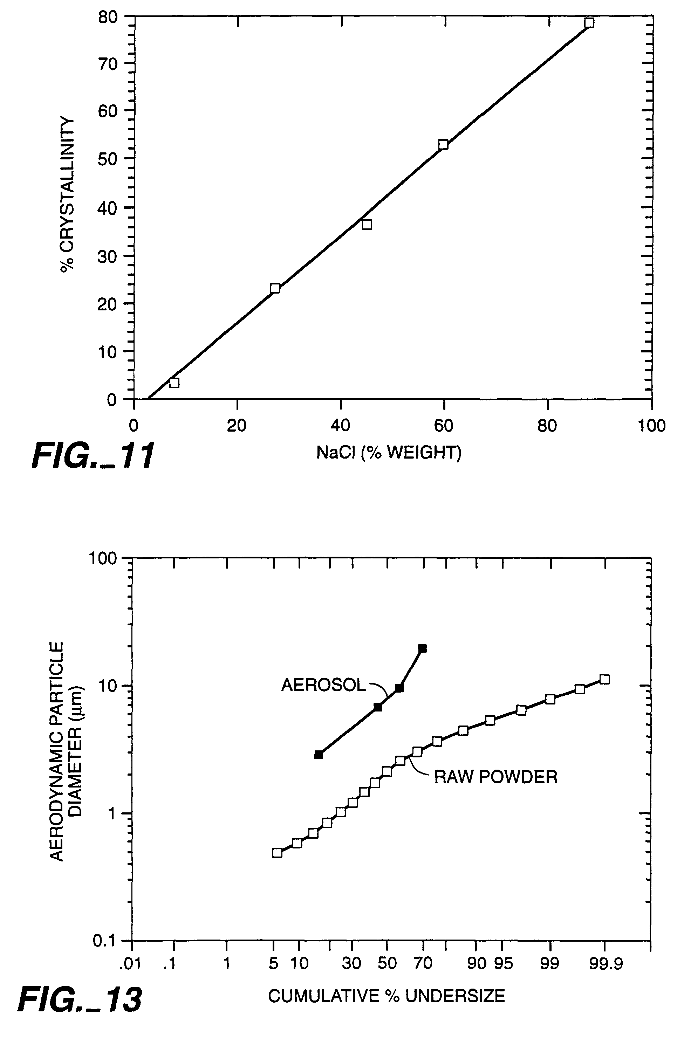

The effect of NaCl content on the redispersion of the rhDNase powders is illustrated in Figure 10. The formulations all have similar particle size distributions with median diameters of 2.7 - 3.3 µm (span from laser diffraction 1.04 - 1.63). A linear plot was found: the higher the salt content, the better the redispersion.

### Effect of Crystallinity And Total Water Content

Crystallinity is a measure of the presence of crystalline matter in the powders. In Figure 11, a linear relationship was obtained between the NaCl content in the formulations and their crystallinity. Consequently, there is a correlation between the powder redispersibility and crystallinity. The relationship between the total water content (Table 1) and redispersibility is not linear, suggesting a possible influence from other physicochemical variables (e.g., the surface adsorbed moisture component rather than the total water content).

### Effect of Particle Morphology

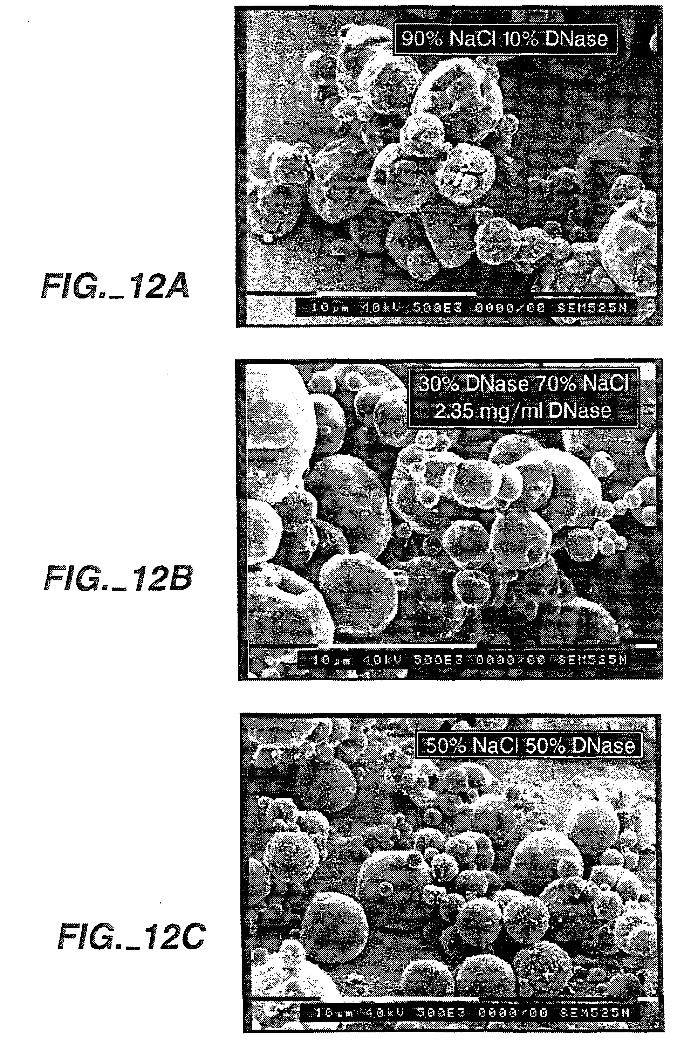

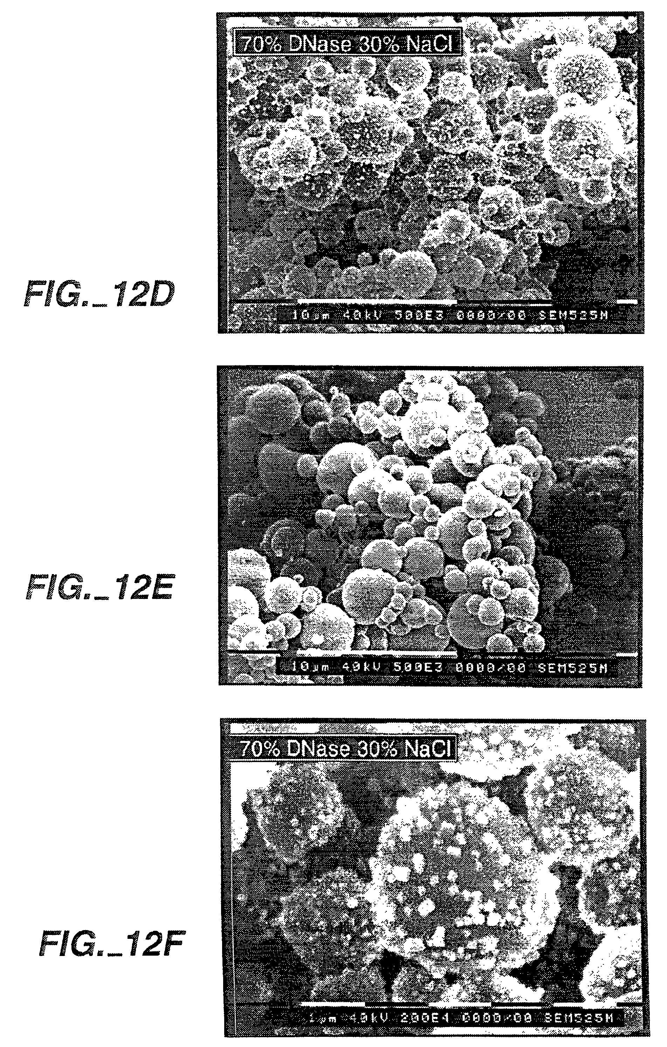

Figures 12A-12F show that as the NaCl content increased, the morphology changed from spheres with very smooth surface textures, to spheres with surfaces having numerous cubic salt crystals, to faceted spheres and finally to spheres of agglomerated salt crystals. A series of morphological features was thus observed. The macroscopic shape is not important since the faceted and smooth spheres were quite similar, yet their redispersions were very different.

### Spray Dried Powders of rhDNase with Sugars

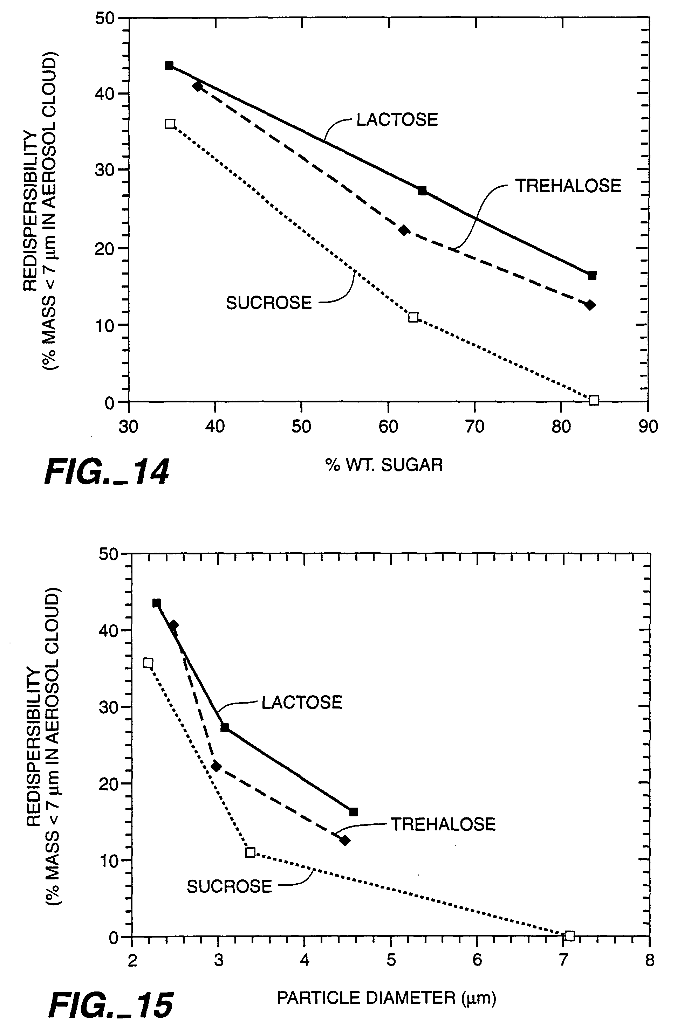

Figure 13 compares, as an example, the particle size distribution of the original powder and the aerosol clouds generated by the Rotahaler for rhDNase powders formulated with 40% lactose. As in the NaCl formulations, it indicates that the size distribution of the original particles is not recovered in the aerosol.

### Effect of Types of Sugars

In Figure 14, rhDNase particles containing lactose were better dispersed than those containing sucrose or trehalose. The highest redispersibility was >40% which is quite comparable to those of the salt formulations. For a given sugar, the redispersibility decreases with increasing sugar content. As the sugar content increased, however, the particle size also increased (the higher sugar % gave larger median size, even though the total solute content in the spraying solutions had been kept at the same initial level). Thus, the change in the powder performance is also partly due to the change of particle size. This is very different from the behavior of the NaCl formulations. For a given sugar, the redispersibility declines from a median particle size of around 2 µm, indicating that the maximum occurs at particle size ≤ 2 µm (Figure 15). Usually fine particles are quite sticky and therefore not dispersed well (Figures 9A and 9B). Fine sugar particles disperse quite well.

### Effect of Crystallinity, Water Content and Particle Morphology

Unlike the NaCl powders which showed distinctive crystallinity due to the salt, none of the sugar powders were crystalline as measured by X-ray powder diffraction. The broad hump observed for the sugar powders is a typical feature of amorphous materials. Therefore, the redispersibility of the sugar powders is not related to crystallinity.

There was no systematic change in redispersibility with water content (Table 2). For a given sugar, the difference in the water content of the powders with different sugar content was only 1 to 2%. The same small difference in moisture was obtained for the three different sugars at similar sugar concentration. Moisture sorption isotherms indicate that crystallization of the Dnase powders occurs at relatively high humidity (about 70% to 85% or more), making it desirable to manufacture and store the powders under conditions of relatively low humidity.

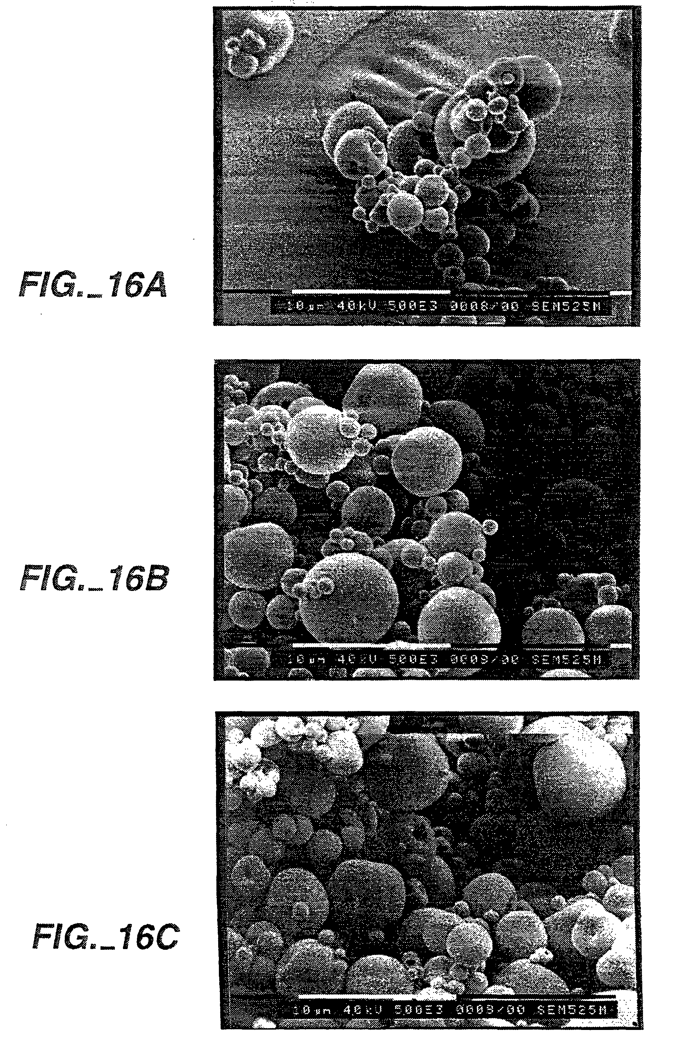

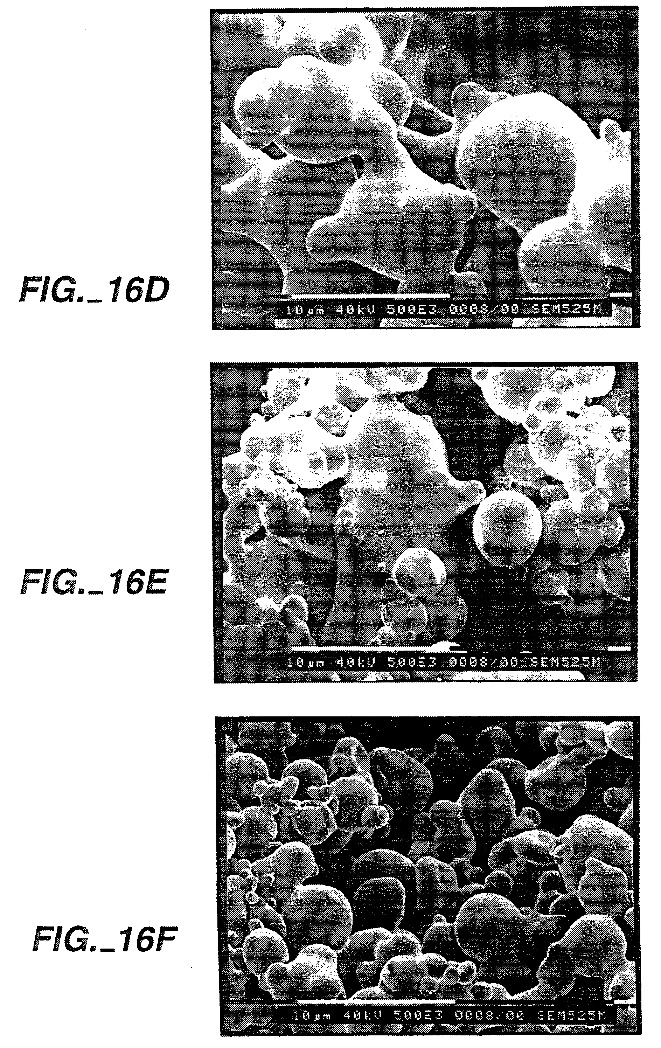

When examined by the SEM, all the sugar formulated rhDNase powders looked alike with very smooth surface textures (Figures 16A-16F) which is also in sharp contrast to the NaCl particles. However, when the sugar content was very high, i.e. 90%, the particle morphology looked really irregular and the size was very large (Figures 16D-F), both of these features explained the much lower redispersibility of these powders. The integrity of the formulations is evidenced by the IEF results in Figure 17.

### Study on Mannitol

Mannitol was attempted at 30% content only. Like other powders, the particles were smooth spheres under SEM. The powder, however, also showed distinct behavior: low moisture content (3.72%) and redispersibility (8.6%) with a larger median particle size (6.5 µm). In addition, the product was crystalline (due to the presence of mannitol).

### Stability

In all studies, the spray-dried powder products showed no significant increase in deamidation. The stability in terms of changes in aggregation and enzymatic activity during storage at various temperatures is shown in the Tables 3-6. It is apparent that the sugars impart good long-term stability on rhDNase powders.

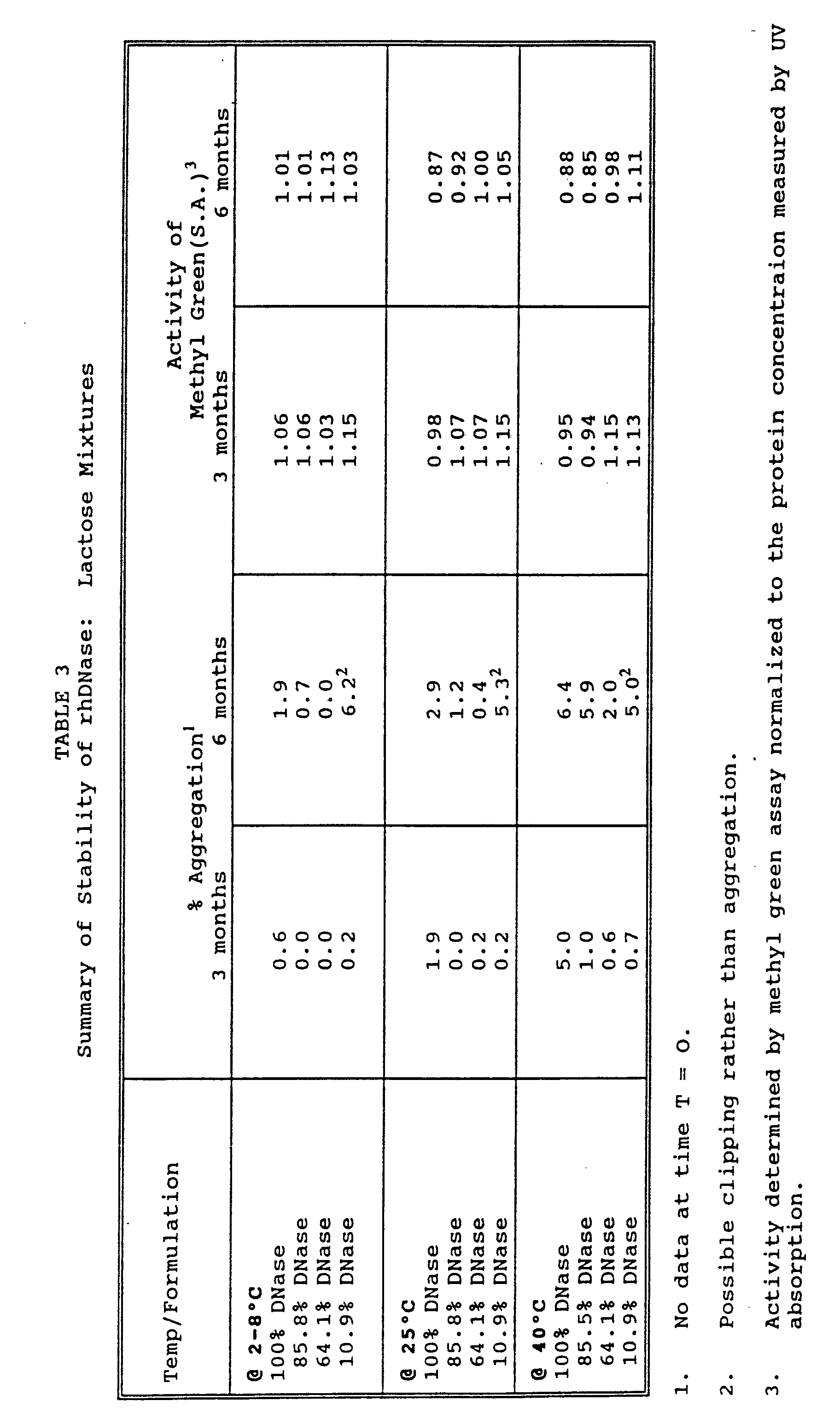

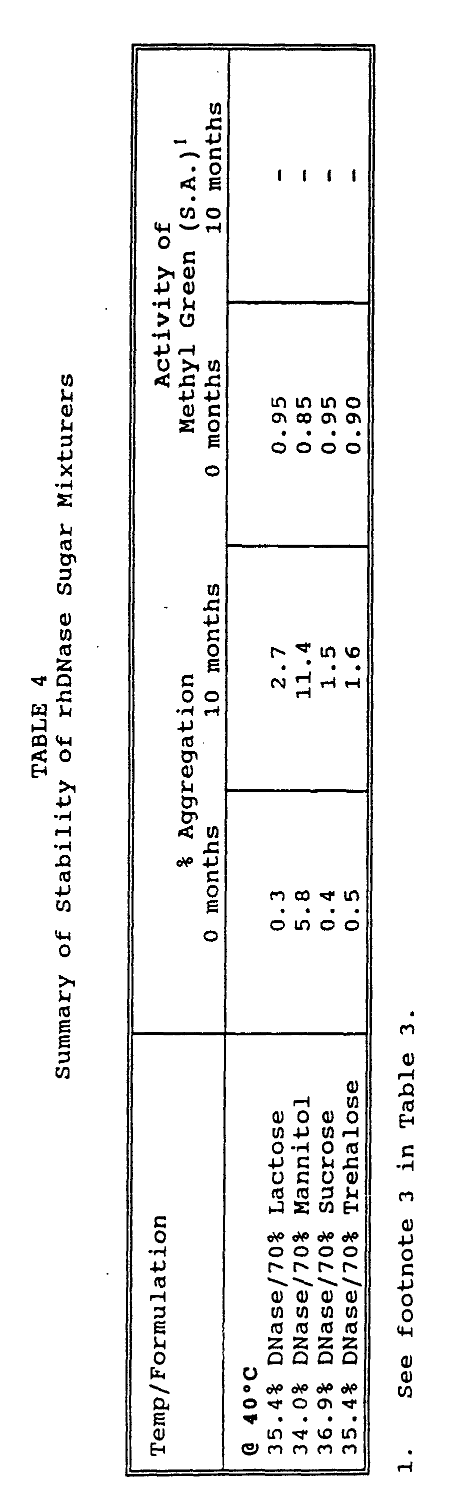

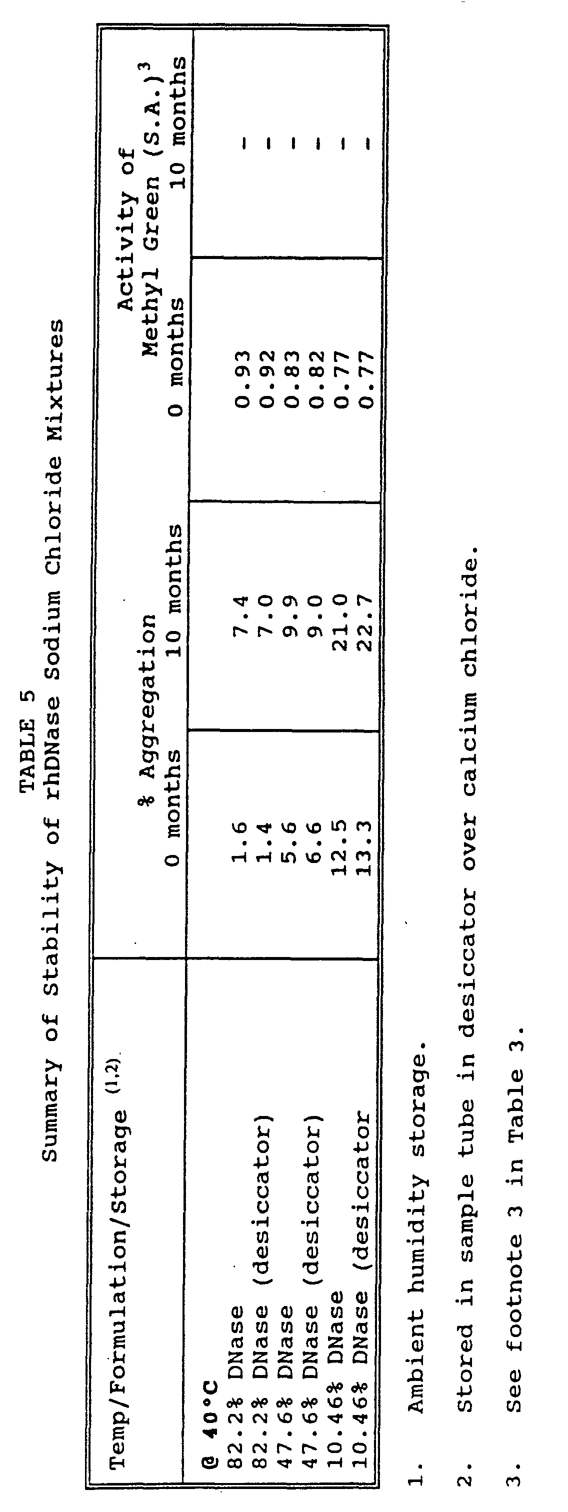

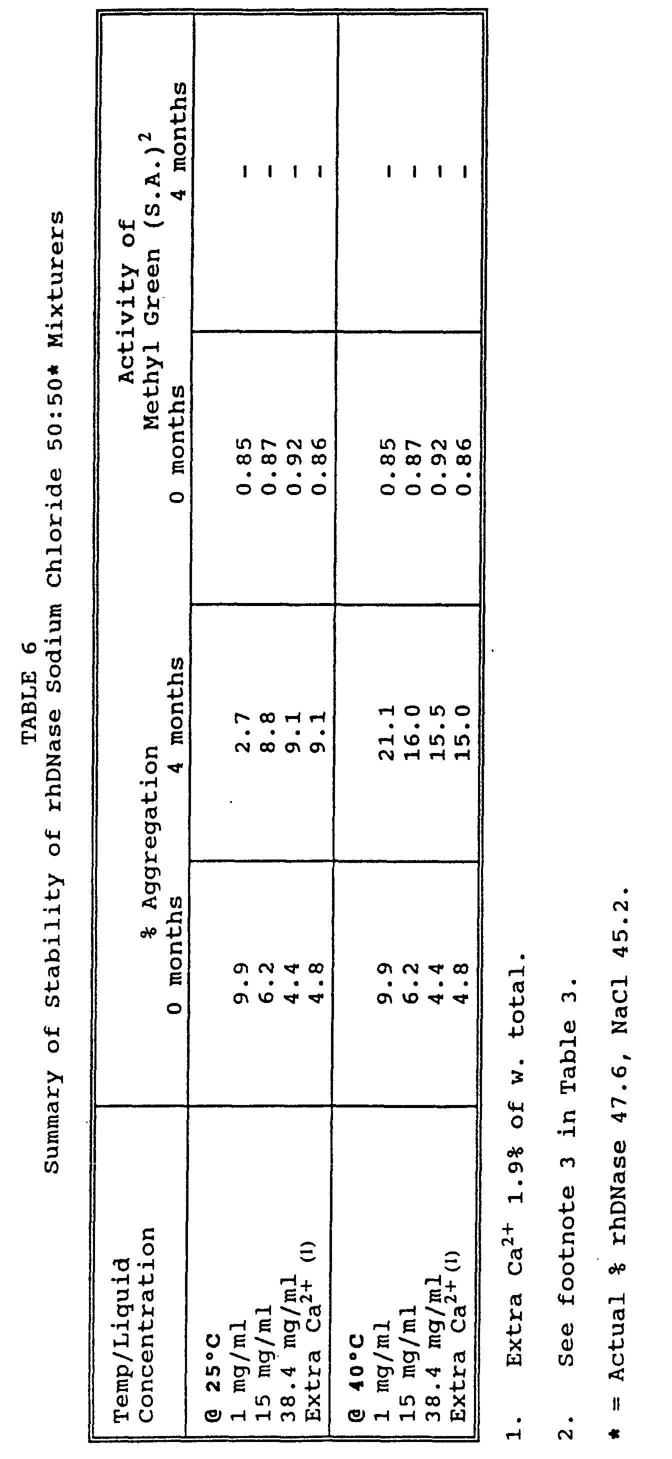

### References

Hatch, T. F. and Gross, P., Pulmonary Deposition and Retention of Inhaled Aerosol, Academic Press, New York, 1964.

Ganderton, D., The generation of respirable clouds from coarse powder aggregates, J. Biopharm. Sci. 3, 101-105 (1992).

Masters, K., Spray Drying Handbook, 4th edition, Wiley & Sons, 1985.

Franks, F., Hatley, R. H. M. and Mathias, S. F., Materials science and the production of shelf-stable biologicals, BioPharm 4(9), 38-42 & 55 (1992).

Bell, J. H., Hartley, P. S. and Cox, J. S. G., Dry powder aerosols I: a new powder inhalation device, J. Pharm. Sci. 60, 1559-1564 (1971).

Vidgren, M., Karkkainen, A., Karjalainen, P., Paronen, P. and Nuuyinen, J., Effect of powder inhaler design on drug deposition in the respiratory tract, Int. J. Pharm. 42, 211-216 (1988).

Kassem, N. M., Ho, K. K. L. and Ganderton, D., The effect of air flow and carrier size on the characteristics of an inspirable cloud, J. Pharm. Pharmacol. 41 (Suppl.) 14P (1989).

Kassem, N. M. and Ganderton, D., Influence of carrier surface on the characteristics of powder aerosols, J. Pharm. Pharmacol. 42 (Suppl.) 11P (1990).

## Claims

1. A method for the preparation of a pharmaceutically acceptable formulation comprising DNase which comprises:
a) spray-drying a liquid composition comprising a compatible excipient and DNase as active principle and
b) collecting the spray-dried product of step a) as a dispersible powder containing DNase in biologically active form.

2. The method according to claim 1 wherein said spray-dried product is in the form of an aerosol suitable for administration via an aerosolization device into the lung of an individual.

3. The method according to claim 1 wherein said spray-drying step is conducted at an inlet temperature such that formed droplets have a temperature not exceeding the denaturation temperature of DNase.

4. The method according to claim 1 wherein said liquid composition has a concentration of DNase from about 1 to about 80 milligrams per millilitre.

5. The method according to claim 1 wherein said DNase-containing powder contains less than about 4% of aggregated form of DNase.

6. The method according to claim 1 wherein said liquid composition comprises NaCl as excipient in an amount of from about 10% to about 90% of the solids content.

7. The method according to claim 1 wherein said liquid composition comprises sugar as excipient in an amount from about 10% to about 90% of the solids content.

8. A pharmaceutically acceptable formulation comprising a spray-dried DNase as active principle and a compatible excipient, said formulation containing DNase in biologically active form, wherein less than about 4% of the active DNase principle is in aggregated form, wherein said product is dispersible and consists substantially of particles of 1-6 microns diameter.

9. A pharmaceutically acceptable formulation according to claim 8 in aerosol form.

10. A pharmaceutically acceptable formulation according to claim 8 wherein said DNase is present therein in an amount of form 0.01 to 1 milligrams per milligram of formulation.

## Patentansprüche

1. Verfahren zur Herstellung einer pharmazeutisch akzeptablen Formulierung umfassend DNase, welches Verfahren umfaßt:
a) Sprühtrocknen einer flüssigen Zusammensetzung umfassend einen kompatiblen Arneimittelhilfsstoff sowie DNase als wirksamer Bestandteil und
b) Sammeln des sprühgetrockneten Produktes aus der Stufe a) als ein dispergierbares Pulver, welches DNase in biologisch aktiver Form enthält.

2. Verfahren nach Anspruch 1, worin das sprühgetrocknete Produkt die Form eines Aerosols hat, welches zur Verabreichung über eine Vernebelungseinrichtung in die Lunge eines Individs geeignet ist.

3. Verfahren nach Anspruch 1, worin die Sprühtrocknungsstufe bei einer solchen Eingangstemperatur durchgeführt wird, daß die Temperatur der geformten Tröpfchen die Denaturierungstemperatur der DNase nicht übersteigt.

4. Verfahren nach Anspruch 1, worin die flüssige Zusammensetzung eine DNase-Konzentration von etwa 1 bis etwa 80 Milligramm pro Milliliter aufweist.

5. Verfahren nach Anspruch 1, worin das DNase-enthaltende Pulver weniger als etwa 4 % DNase in aggregierter Form enthält.

6. Verfahren nach Anspruch 1, worin die flüssige Zusammensetzung NaCl als Arneimittelhilfsstoff in einer Menge von etwa 10 % bis etwa 90 % des Feststoffanteiles enthält.

7. Verfahren nach Anspruch 1, worin die flüssige Zusammensetzung Zucker als Arneimittelhilfsstoff in einer Menge von etwa 10 % bis etwa 90 % des Feststoffanteiles umfaßt.

8. Pharmazeutisch akzeptable Formuliering umfassend eine sprühgetrocknete DNase als wirksamer Bestandteil sowie einen kompatiblen Arneimittelhilfsstoff, wobei die Formulierung DNase in einer biologisch aktiven Form enthält, bei der weniger als 4 % des wirksamen DNase-Bestandteils in aggregierter Form vorliegt, und wobei das Produkt dispergierbar ist und im wesentlichen aus Partikeln mit einem Diameter von 1 bis 6 Mikrometern besteht.

9. Pharmazeutisch akzeptable Formulierung nach Anspruch 8 in Aerosol-Form.

10. Pharmazeutisch akzeptable Formulierung nach Anspruch 8, worin die DNase in einer Menge von 0,01 bis 1 Milligramm pro Milligramm der Formulierung vorliegt.

## Revendications

1. Méthode de préparation d'une formulation pharmacologiquement acceptable comprenant de la DNase, la méthode comprenant:
a) séchage par pulvérisation d'une composition liquide comprenant un excipient compatible ainsi que de la DNase comme principe actif et
b) collecte du produit séché par pulvérisation de l'étape a) comme une poudre dispersible contenant de la DNase dans une forme biologiquement active.

2. Méthode selon la revendication 1, dans laquelle ledit produit séché par pulvérisation a la forme d'un aérosol convenable pour l'administration par un dispositif d'aérosolisation dans le pumon d'un individu.

3. Méthode selon la revendication 1, dans laquelle ladite étape de séchage par pulvérisation est réalisée à une température d'entrée telle que les goutelettes formées aient une température qui ne dépasse pas la température de dénaturation de la DNase.

4. Méthode selon la revendication 1, dans laquelle ladite composition liquide a une concentration de DNase d'environ 1 à environ 80 milligrammes par millilitre.

5. Méthode selon la revendication 1, dans laquelle ladite poudre contenant la DNase contient moins d'environ 4 % de DNase sous forme agrégée.

6. Méthode selon la revendication 1, dans laquelle ladite composition liquide comprend du NaCl comme excipient dans une quantité d'environ 10 % à environ 90 % de la teneur en matières solides.

7. Méthode selon la revendication 1, dans laquelle ladite composition liquide comprend du sucre comme excipient dans une quantité d'environ 10 % à environ 90 % de la teneur en matières solides.

8. Formulation pharmacologiquement acceptable comprenant une DNase séchée par pulvérisation comme principe actif ainsi qu'un excipient compatible, ladite formulation contenant de la DNase dans une forme biologiquement active, dans laquelle moins d'environ 4 % du principe actif DNase est sous forme agrégée, et ledit produit étant dispersible et consistant substantiellement de particules ayant un diamètre de 1 à 6 micromètres.

9. Formulation pharmacologiquement acceptable selon la revendication 8 sous forme d'aérosol.

10. Formulation pharmacologiquement acceptable selon la revendication 8, dans laquelle ladite DNase est présente dans une quantité de 0,01 à 1 milligrammes par milligramme de formulation.
